# EUROPEAN PATENT APPLICATION

(11) **EP 4 226 885 A1**
(43) Date of publication of application: **16.08.2023**
(21) Application number: 22305140.0
(22) Date of filing: 09.02.2022
(51) Int. Cl.: A61B 34/10, G16H 20/40, G16H 30/40, G16H 50/70

(54) **METHOD FOR SIMULATING A DEVICE DEPLOYMENT**

(71) Applicant: Predisurge, 42000 Saint-Etienne (FR); Institut Mines-Télécom (IMT), 91123 Palaiseau (FR); Association Pour La Recherche Et Le Développement De Méthodes Et Processus Industriels "Armines", 75272 Paris Cedex 06 (FR)
(72) Inventor: BISIGHINI, Beatrice, 42000 Saint-Etienne (FR); AGUIRRE, Miquel, 42023 Saint-Étienne (FR); PIERRAT, Baptiste, 75272 Paris (FR); AVRIL, Stéphane, 42023 Saint-Étienne (FR); PERRIN, David, 42000 Saint-Etienne (FR)
(74) Representative: Icosa

(57) **Abstract**

The invention relates to a method for training a machine learning system, comprising:
- based on at least one image dataset (I) representing at least one portion of the hollow structure:
• calculating (S10) a signed distance field of each portion; and
• calculating (S11) at least one geometrical parameter of each portion;

- generating (S20) a deployed in-use representation of the device by:
• computing contact forces between the device and each portion based on the signed distance field; and
• applying (S202) said contact forces to a geometrical representation of the device;

- training (S30) the machine learning system (ML) with each calculated geometrical parameter as an input and the corresponding deployed in-use representation as an associated target output, the obtained trained machine learning system being configured to receive as input at least one geometrical parameter and provide as output the deployed in-use representation of the device.

## Description

### FIELD OF INVENTION

The present invention relates to a computer-implemented method for training a machine learning system configured for generating an optimal deployed configuration of a device inside a hollow structure of a subject for surgical planification.

The invention further concerns a corresponding computer program product and device.

The invention relates to the fields of surgical planning and surgical navigation.

### BACKGROUND OF INVENTION

Expanding, or deployable, devices are used in a variety of conditions in which the patency of a lumen of the human body needs to be maintained or restored.

These devices comprise for instance dilating balloons and expandable stents, as well as their combination (*e.g.*, in angioplasty). Balloons are typically inserted in a narrowed lumen (for instance, a stenosed blood vessel), inflated with a pump, then deflated and removed, whereas stents are usually permanent structures which remain in place after insertion and expansion.

Expandable devices are crucial to ensure the patency of both vascular and non-vascular structures. In order to ensure their efficiency and the patient's safety, an accurate planning of their insertion, as well prediction of their expansion *in situ,* is required.

To this end, medical images are not sufficient, because they provide limited visibility.

Several techniques have been developed to simulate the deployment of expandable devices in a pre-operative setting. Nevertheless, these techniques are complex and time-consuming, hence not adapted to provide real time information.

Even though some attempts have been made to improve these methods, the proposed solutions typically involve over-simplifications of the device and/or the anatomy, which are not compatible with the standards of personalized medicine and precision medicine. Moreover, these simplifications may result in under- or over-estimation of the risks associated with a surgical intervention.

A purpose of the invention is to provide an expandable device deployment simulation method that is fast, reliable and accurate.

### SUMMARY

To this end, the present invention is a computer-implemented method of the aforementioned type, comprising:
- based on at least one image dataset representing at least one portion of the hollow structure:
   - calculating a signed distance field of the at least one portion of the hollow structure; and
   - calculating at least one geometrical parameter of the at least one portion of the hollow structure;
- generating a deployed in-use representation of the device by:
   - computing contact forces between the device and the at least one portion of the hollow structure based on the signed distance field; and
   - applying said contact forces to a geometrical representation of the device;
- training the machine learning system with each calculated geometrical parameter as an input variable and the corresponding deployed in-use representation of the device as an associated target output, so as to obtain a trained machine learning system configured to receive as an input at least one geometrical parameter and provide as output the deployed in-use representation of the device to be used in a method for generating an optimal deployed configuration of a device for surgical planification.

Indeed, the signed distance field and the at least one parameter are **directly derived from the image dataset,** thereby ensuring the patient-specificity of the deployed in-use representation of the device thus obtained.

Moreover, the introduction of spatial errors intrinsic to mesh-generating algorithms is avoided because the **contact forces are computed from the signed distance field,** which is directly derived from images.

Furthermore, the generation of the optimal deployed configuration C of the device D by the trained machine learning system does not require the execution of time-consuming steps, such as: iterative solving algorithms, execution of finite element methods, computation of the signed distance field. All these steps are previously performed.

According to other advantageous aspects of the invention, the method comprises one or more of the following features, taken alone or in any possible combination:
the hollow structure is a blood vessel, a fistula, or a non-vascular tract of the human body;
the method further comprises segmenting the at least one image dataset to obtain a segmented image dataset, and wherein the at least one geometrical parameter and/or the signed distance field are calculated based on the segmented image dataset;
the segmenting comprises applying a level set segmentation to the image dataset;
the method further comprises extracting, from the segmented image dataset, a three-dimensional surface representing the hollow structure, preferably using a marching cube algorithm;
the segmenting comprises an initialization phase for obtaining a zero-level set and an evolution phase, said initialization phase including:
   - selecting at least two points in the at least one image dataset;
   - performing a colliding front algorithm using the at least two points as seed points;
the segmented image dataset comprises a set of points or a set of curves, and the calculation of the at least one geometrical parameter comprises:
   - applying a point set registration to the set of points; or
   - applying a curve-based parametrization to the set of curves;
the geometrical representation of the device comprises beam elements and the applying phase comprises applying a corotational formulation to the beam elements;
the method further comprises, prior to the training, reducing an order of the deployed in-use representation of the device so as to obtain a reduced order representation of the deployed device, and wherein during the training the machine learning system is trained with the calculated geometrical parameter as an input variable and a subset of features of the reduced order representation as an associated target output;
the reducing comprises: applying a proper orthogonal decomposition algorithm to the deployed in-use representation of the device;
the at least one geometrical parameter is a centerline, a center, or a radius of the hollow structure;
the method further comprises: generating a deformed representation of the device, the deformed representation being representative of a configuration of the device after implantation inside the hollow structure of the subject, the geometrical representation of the device to which contact forces are applied being said deformed representation.

The **segmentation** of the image dataset advantageously allows to obtain, with a single operation, both the signed distance field and the geometrical parameter.

**Level set segmentation** relies on spatial variations in image intensity rather than on absolute intensity. Accordingly, level set segmentation is robust, and less sensitive to changes in the intensity of the hollow structure in the image dataset *(e.g.,* due to artifacts, contrast bolus dynamics, or partial volume effects). Moreover, level set segmentation is highly reproductible because the number of user-dependent steps is minimized (*e.g.*, manual definition of intensity thresholds), and it is based on criteria which can be univocally defined *(e.g.,* gradient of an image intensity).

By calculating at least one **geometrical parameter** on the basis of the segmented image dataset, it is possible to obtain a parameter which is independent of the type and shape of the organ or of the hollow structure. In other words, the same parameter may be used to describe different anatomical regions or organs of the subject.

For instance, a **centerline** is particularly useful to parametrize a hollow structure having a dimension larger than the other dimensions, such as a vessel, or a tubular tract of the body. The **center and/or the radius** are particularly useful to parametrize a hollow structure having similar dimensions, such as aneurism.

The segmentation step typically comprises an initialization phase and an evolution phase. An initialization phase based on **colliding fronts** is particularly advantageous for obtaining a zero-level set of a hollow structure having a tubular shape, such as a blood vessel. Indeed, in this case a first point may be selected on one extremity of the hollow structure, and a second point may be selected on the opposite extremity of the hollow structure.

This **three-dimensional surface** of the hollow structure may be used for visualization purposes. Optionally, it may be used to derive one or more geometrical parameters. Advantageously, the three-dimensional surface is obtained from the signed distance field, and not the contrary. Therefore, the loss of accuracy introduced by surface-generation algorithms (such as marching cubes) is controlled such that it affects only the three-dimensional surface generation, and not the signed distance field.

**Beam elements** allow to represent a wire mesh undergoing large, non-linear deformations. Therefore, they are particularly adapted for representing devices such as knitted, braided or woven stents. For instance, beam elements could be modelled using a corotational formulation. The corotational formulation advantageously allows to simulate large, non-linear deformations, as it may be the case for a device D comprising a wire mesh *(e.g.,* a braided stent). Moreover, the corotational formulation also allows to reduce computational load.

Inputting the **reduced order representation** into the machine learning system allows to obtain a trained machine learning system capable of providing a deployed in-use representation of the device in an amount of time which is independent of the order, or dimensions, of said representation. Indeed, on the basis of the relationship between reduced bases and the corresponding geometrical parameter, the trained machine learning system may derive the deployed in-use from a fixed number of reduced bases.

Moreover, the order reduction allows to reduce the computational cost needed to provide a representation of the deployed device, while controlling the eventual loss of accuracy.

For instance, a **proper orthogonal decomposition** allows to reduce the order of the deployed in-use representation of the device into a set of reduced bases.

The **crimping and the deformation** steps allow to provide an initial configuration for the simulation step which is close to the real position of the device right after implantation. Therefore, the deployed in-use representation of the device thus obtained closely reproduces the real deployed configuration of the same.

The **deformation** based on the at least one geometrical parameter ensures that the geometrical representation fits the hollow structure (for instance, said crimped representation may be deformed so that it follows the centerline of the hollow structure), as it would be the case for a flexible device implanted inside an organ.

By using a **machine learning system trained** according to anyone of the embodiments described hereinabove, it is possible to accurately predict the deployed configuration of the device in real time. More precisely, the deployed representation is provided only based on geometrical parameters derived from images, without the need to perform operations such as: assess mechanical behaviors with conventional finite element methods, generate meshes of the organ, solve differential equations describing the contacts between the device and the walls of the organ, and the like.

Moreover, the machine learning system is trained with inputs obtained via numerical simulation of the stent deployment, rather than image-based simulation which do not take into account the mechanical behavior of the device.

The present invention also relates to a non-volatile computer storage medium comprising instructions which, when executed by a computer, cause the computer to carry the steps of the method according to any one of the embodiments described hereabove.

Furthermore, the invention relates to a computer-implemented method for generating an optimal deployed configuration of a device inside an organ of a subject for surgical planification, the method comprising:
- calculating at least one geometrical parameter of a hollow structure of the subject based on an image dataset comprising at least the hollow structure;
- generating an optimal deployed configuration of the device by inputting the calculated at least one geometrical parameter into a machine learning system trained according to the method as defined above.

Furthermore, the present invention relates to a device for training a machine learning system configured for generating an optimal deployed configuration of a device inside a hollow structure of a subject for surgical planification, the device comprising a processing unit configured to:
- based on at least one image dataset representing at least one at least one portion of the hollow structure:
   - calculate a signed distance field of the at least one portion of the hollow structure; and
   - calculate at least one geometrical parameter of the at least one portion of the hollow structure;
- generate a deployed in-use representation of the device by:
   - compute contact forces between the device and the at least one portion of the hollow structure based on the signed distance field; and
   - apply said contact forces to a geometrical representation of the device;
- train the machine learning system with each calculated geometrical parameter as an input variable and the corresponding deployed in-use representation of the device as an associated target output, so as to obtain a trained machine learning system configured to receive as an input at least one geometrical parameter and provide as output the deployed in-use representation of the device to be used in a method for generating an optimal deployed configuration of a device for surgical planification.

Furthermore, the present invention relates to a device for generating an optimal deployed configuration of a device inside an organ of a subject for surgical planification, the device comprising a processing unit configured to:
- calculate at least one geometrical parameter of a hollow structure of the subject based on an image dataset comprising at least the hollow structure;
- generate an optimal deployed configuration of the device by inputting the calculated at least one geometrical parameter into a machine learning system trained according to the method of as defined above.

### BRIEF DESCRIPTION OF THE DRAWINGS

The invention will be better understood with the attached figures, in which:
- **figure 1** is a schematic representation of a first embodiment of a device 1 for surgical planification according to the present invention;
- **figure 2** is a frontal view showing a device D configured to be implanted in a hollow structure H, the device D being represented in an unrestrained deployed configuration and in a collapsed configuration;
- **figure 3** is an image showing a three-dimensional surface representative of an exemplary hollow structure H and a corresponding geometrical parameter P. More precisely, **figure 3A** illustrates the three-dimensional surface of a blood vessel and the blood vessel center line, **figure 3B** further illustrates a geometrical representation R1 of the device D inside the hollow structure H of figure 3A, and **figure 3C** illustrates the corresponding deployed in-use representation R2 of said device D;
- **figure 4** is an image schematically illustrating an exemplary image dataset I comprising a hollow structure H;
- **figure 5** is an image schematically illustrating an SDF generated from the image dataset of figure 4;
- **figure 6** is a flowchart representing a first embodiment of a method for training a machine learning system according to the invention;
- **figure 7** is an image illustrating a geometrical representation R1 of the device D of figure 2 to which contact forces are applied;
- **figure 8** is a flowchart representing a method for training a machine learning system of the invention according to a first embodiment;
- **figure 9** is a schematic representation of a device 1 for surgical planification according to the present invention according to a second embodiment.

### DETAILED DESCRIPTION

For the purpose of illustrating, the invention is shown in the preferred embodiments. It should be understood, however that the application is not limited to the precise arrangements, structures, features, embodiments, and aspect shown. The drawings are not drawn to scale and are not intended to limit the scope of the claims to the embodiments depicted.

### Device 1 for surgical planification

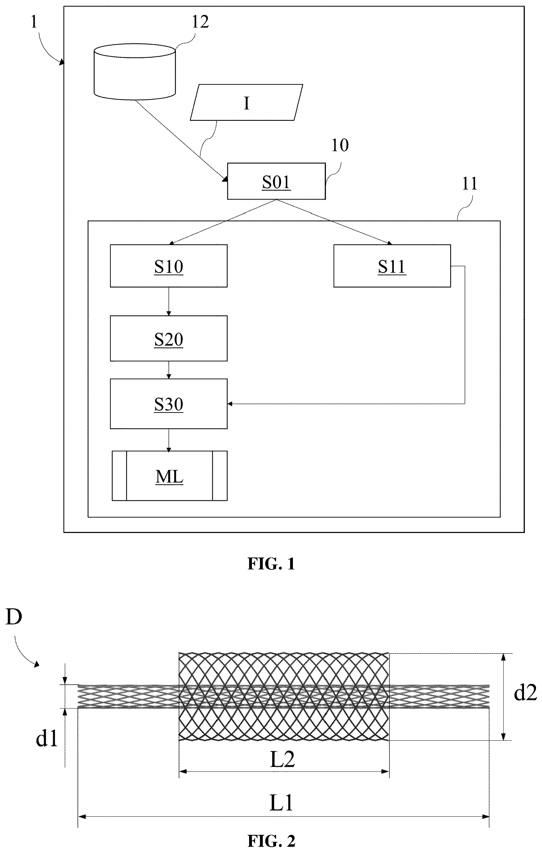

A device 1 for surgical planification according to the invention is shown on **figure 1**.

More precisely, the device 1 is configured to train a machine learning system ML configured to generate an optimal deployed configuration of a device D inside a hollow structure H of a subject.

The hollow structure H may be any lumen or cavity of the human body including, without limitation: a blood vessel, such a vein, an artery or a fistula, a non-vascular structure, such as a respiratory tract *(e.g.,* trachea, bronchi, esophagus), a gastrointestinal tract *(e.g.,* the colon), a biliary tract, a renal tract, or a urinary tract, and the like.

All these hollow structures H are adapted to receive a device D having a deployed configuration.

An example of a device D having a collapsed, or crimped, configuration (with diameter d1 and length L1) and a deployed, or expanded, configuration (with diameter d2 and length L2) is shown on **figure 2**. Particularly, this device D is a braided stent.

Stents are tubular devices allowing to maintain or restore the patency of a lumen *(e.g.,* to restore blood flow in a vessel, to impede intraluminal growth of neoplastic tissue). They may be made of a mesh of fibers or wires (*e.g.*, braided stents, knitted stents, woven stents).

The deployed configuration (d2, L2) of the stent of **figure 2** is an unrestrained deployed configuration.

More precisely, the term *"unrestrained"* refers to a configuration of a device D which is only affected by intrinsic properties of said device D *(e.g.,* radius, material, Young modulus, etc.), and not by extrinsic properties (*e.g.*, presence, shape, mechanical properties of surrounding tissues).

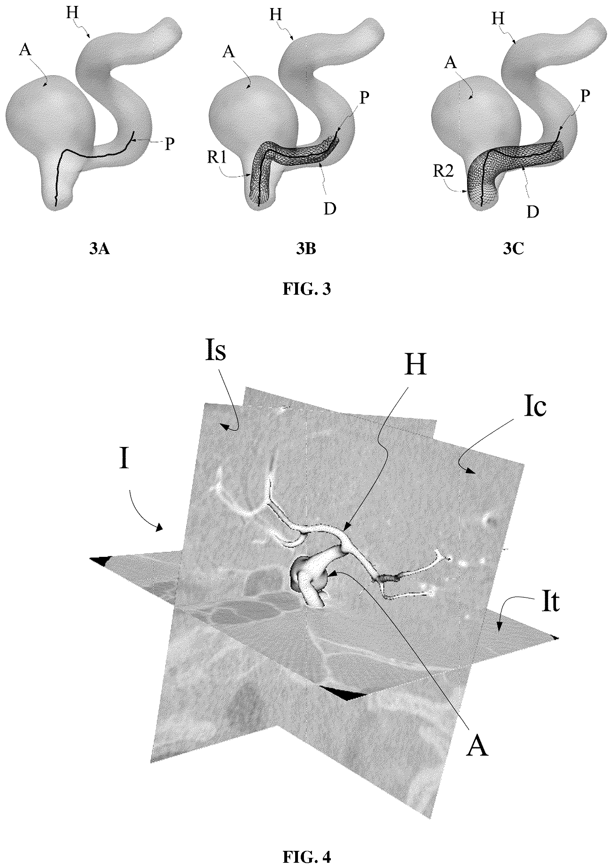

**Figure 3A** illustrates one exemplary hollow structure H (blood vessel), and figures **3B** and **3C** respectively illustrate a collapsed and an expanded configuration of the device D (in this case, a braided stent) when implanted inside the hollow structure H. The bulge on the blood vessel of **figure 3** is an aneurysm A.

The deployed configuration of **figure 3C** is an in-use deployed configuration.

Further description of the device 1 will now be provided.

As shown on **figure 1****,** the device 1 includes an input 10 for receiving S01 at least one image dataset I, and a processing unit 11 configured to train a machine learning system ML based on each received image dataset I.

### Input 10

As shown in **figure 1****,** the device 1 of the present invention may comprise an input 10 adapted to receive, during a receiving step S01, at least one image dataset I. The image dataset I comprises at least one portion of the hollow structure H.

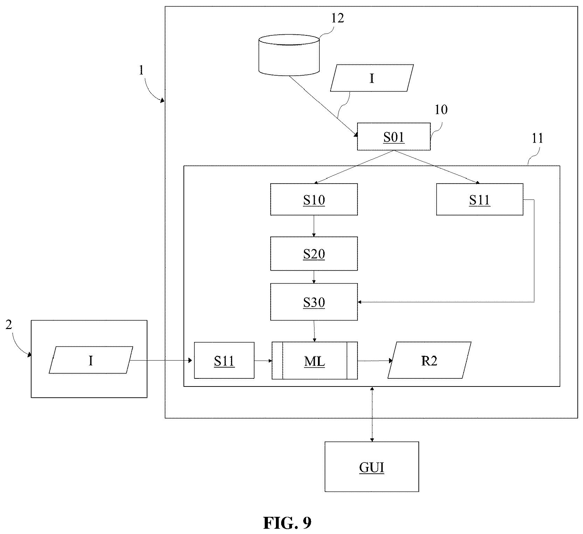

For instance, the image dataset I may be stored in one or more local or remote database(s) 12. Alternatively, or in addition, the input of the device 1 may be directly connected to an imaging apparatus 2 **(****figure 9****).**

The image dataset I preferably comprises medical images such as magnetic resonance (MR) images, computed tomography (CT) images or rotational angiography (RA) images. The images may be acquired with or without contrast enhancement.

One exemplary image dataset I is schematically illustrated in **figure 4****.** In this example, the hollow structure H includes a cerebral artery (which displays an aneurism A) and its branches, and the image dataset I is a magnetic resonance angiogram (or MRA) comprising transverse, sagittal, and coronal sections (or slices) It, Is, Ic.

For intelligibility purposes, only three orthogonal sections of the image dataset I are illustrated in **figure 4****,** each of which comprises at least one portion of the aneurysm.

### Processing unit 11

As mentioned previously, the device 1 comprises a processing unit 11.

The processing unit 11 is configured to implement a series of steps which will now be described in more details. The main steps comprise: a first calculation step S10, a second calculation step S11, a generating step S20 and a training step S30 **(****figure 1****).**

### Calculation S10 of SDF

During a first calculation step S10, the processing unit 11 is configured to calculate a signed distance field (SDF) of the at least one portion of the hollow structure H on the basis of the image dataset I.

The SDF is a volumetric grid wherein each voxel is associated with the value of the distance from a target surface. The value of the SDF is zero on the three-dimensional surface (which forms the target surface), and it is negative inside the three-dimensional surface and positive outside of the three-dimensional surface, or *vice versa.*

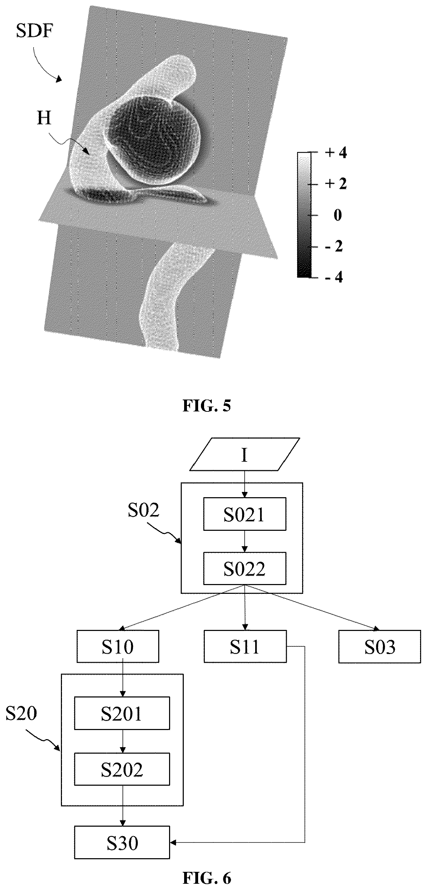

One exemplary SDF is shown in **figure 5****.** The color bar illustrates the values of the SDF (units: mm).

More precisely, two orthogonal sections of the SDF are herein illustrated. For intelligibility purposes, a three-dimensional surface of the hollow structure H is also illustrated.

Preferably, the processing unit 11 is configured to calculate the SDF from a set of points obtained by segmenting S02 the image dataset I. This segmentation step S02 will later be described.

### Calculation S11 of geometrical parameter(s) P

During a second calculation step S11, the processing unit 11 is configured to calculate at least one geometrical parameter P of the portion of the hollow structure H on the basis of the image dataset I.

The geometrical parameter(s) P and the calculation thereof may be selected depending on the hollow structure H.

For example, the geometrical parameter P may comprise one or more parameter(s) such as: an axis, a centerline **(****figure 3****),** a center **(****figure 3****),** or a radius of the hollow structure H.

For instance, the processing unit 11 may be configured to calculate a center for spherical or quasi-spherical hollow structures H such as an aneurism A. As another example, the processing unit 11 may be configured to calculate a centerline for elongated hollow structures H such as blood vessels.

For instance, the centerline may be calculated as the path defined on Voronoi diagram sheets that minimize the integral of the radius of maximal inscribed spheres along said path.

### Segmentation S02

Preferably, the processing unit 11 is configured to segment the image dataset I, during a segmentation step S02 prior to calculation steps S10, S11, to obtain a segmented image dataset Is. In this case, the processing unit 11 is configured to calculate, during calculation steps S10, S11, the at least one geometrical parameter P and/or the signed distance field SDF based on the segmented image dataset Is.

The segmented image dataset Is includes a set of surfaces or curves comprising points (*e.g.*, corresponding to pixels or voxels having the same intensity).

For instance, said surfaces or curves may comprise points obtained from a segmenting step S02 based on a region growing algorithm from a seed point *(e.g.,* from a user-selected voxel).

One example of segmenting step S02 according to the invention is schematically represented in **figure 6****.**

For instance, during the segmenting step S02, the processing unit 11 may be configured to apply a level set segmentation to the image dataset I. This allows to generate a target surface, or curve, in an implicit form as the isosurface at the zero-level set of a higher-dimension function which is referred to as *"level set function".*

As shown in **figure 6****,** the segmenting step S02 may comprise an initialization phase S021 and an evolution phase S022.

In the initialization phase S021, the processing unit 11 is configured to define an initial curve, or surface inside the hollow structure H in the image dataset I.

For instance, for a level set segmentation, the initial surface of the initialization phase S021 is an initial zero-level set.

Advantageously, in a level set segmentation the initial zero-level set is close to the target surface.

The processing unit 11 is configured to, during the initialization phase S021:
- select at least two points in the image dataset; and
- run a colliding front algorithm using the at least two points as seed points.

This particular initialization phase S021 is preferred for a hollow structure H having a tubular shape, such as a blood vessel. In this case, a first point may be selected on one extremity of the hollow structure H, and a second point may be selected on the opposite extremity of the hollow structure H.

During the evolution phase S022 of the segmenting step S02, the processing unit 11 is configured to deform the predefined curve, or surface (which has been defined inside the hollow structure H during the initialization phase S021) until it reaches the boundaries of the hollow structure H. In this case, the segmented image dataset Is includes the deformed curve or surface obtained.

For instance, in a level set segmentation, the processing unit 11 is configured to deform, during the evolution phase S022, the initial zero level set by applying a set of partial differential equations thereto, so as to obtain the target surface. In this case, the segmented image dataset Is includes the target surface. The processing unit 11 may further be configured to derive, during the first calculation step S10, the SDF based on a relationship between the SDF and the target surface. For instance, the processing unit 11 may be configured to select as SDF said target surface.

Advantageously, the level set segmentation ensures that the segmenting step S02 is robust and highly reproductible since it minimizes the number of user-dependent steps, and it is based on the gradient of intensity, rather than an absolute intensity, of the image dataset I.

However, the processing unit 11 may be configured to implement different initialization and evolution phases S021, S022, for instance depending on the shape of the hollow structure H.

### Extraction S03 of a three-dimensional surface

The processing unit 11 is further configured to extract, during an optional extraction step S03, from the segmented image dataset Is, a three-dimensional surface representing the hollow structure H, as shown in **figure 6****.** For instance, said three-dimensional surface may be extracted using a marching cube algorithm.

The three-dimensional surface representing the hollow structure H may be outputted for visualization purposes.

Examples of said three-dimensional surface are shown in **figures 3** (herein shown as overlapped to the representations R1, R2 of the device D and the geometrical parameter P) and in **figure 4** (herein being shown as overlapped to the image dataset I).

Advantageously, it is the three-dimensional surface which is obtained from the SDF, and not *vice versa.* Therefore, the loss of accuracy introduced by surface-generation algorithms (such as marching cubes algorithms) does not affect the SDF calculation.

### Generation S18 of deformed representation

The processing unit 11 may advantageously be configured to generate, during a generation step S18, a deformed representation of the device D.

The generation S18 of the deformed representation of the device D is better illustrated in **figures 2** and **3**. Said deformed representation may be generated by:
- applying a predetermined displacement (*e.g.*, applying a radial displacement to each node of a mesh) to an unrestrained deployed representation of the device D (e.g., configuration d2, L2 of **figure 2**) to obtain a collapsed, or crimped, representation of the same (e.g., configuration d1, L1 of **figure 2**); and
- deforming the collapsed representation of the device D on the basis of the geometrical parameter P of that hollow portion H (**figure 3A**).

For instance, during said deforming, the processing unit 11 may be configured to deform the collapsed representation of the device D such that it is aligned with a centerline of the hollow portion H.

Different techniques may be used for deforming the collapsed representation of the device D based on the geometrical parameter P, depending on the shape of the device D and/or of the hollow structure H.

For instance, for a tubular device D, the deforming of the collapsed representation may comprise: translating and/or morphing an initially straight centerline of the tubular device **(****figure 2****)** according to a centerline of the hollow structure H obtained during a segmenting step S02 **(figure 3A).**

The deformed representation thus obtained **(figure 3B)** is representative of a configuration of the device D inside the hollow structure H right after implantation.

Preferably, the processing unit 11 is configured to implement a finite element solver to perform this generating step S18.

In this case, the processing unit 11 *(e.g.,* the finite element solver) may be configured to iteratively apply incremental displacements to the unrestrained deployed representation of the device D, until the predetermined displacement is applied. More precisely, at each iteration, the processing unit 11 is configured to apply a predetermined constraint component *(e.g.,* a radial displacement or force) to the nodes of the mesh and to compute the remaining (*i.e.,* unconstrained) components associated to said constraint *(e.g.,* a longitudinal displacement).

Moreover, the processing unit 11 may be configured to iteratively apply incremental deformations to the collapsed representation of the device D, until said collapsed representation is comprised within the hollow structure H. More precisely, at each iteration, the processing unit 11 is configured to apply a predetermined constraint component *(e.g.,* a translation and/or a rotation) to the nodes of the mesh and to compute the remaining *(i.e.,* unconstrained) component associated to said constraint.

In each of the iterations described hereinabove, the processing unit 11 may be configured to input the computed components in the next iteration, in order to obtain updated boundary conditions.

This deformed representation may be used as initial condition in a finite element solver configured to generate, during generating step S20, the deployed in-use representation R2 **(figure 3C)** therefrom.

### Generation S20 of a deployed in-use representation R2

During the generating step S20, the processing unit 11 is configured to generate the deployed in-use representation R2 **(figure 3C)** of the device D based on the previously calculated SDF. To this end, the generating step S20 advantageously includes a contact forces computation phase S201, and a contact forces application phase S202.

The step S20 of generating the deployed in-use representation R2 based on the SDF has several advantages.

First, contact search algorithms *(i.e.,* algorithm to find the closest points to a surface) and mesh-generating algorithms are avoided, thereby allowing to generate S20 the deployed in-use representation R2 rapidly.

Moreover, the introduction of spatial errors, which are intrinsic to mesh-generating algorithms, is avoided.

Furthermore, as the deployed in-use representation R2 is generated based on the SDF, which is in turn directly derived from the image dataset I, the number of intermediate objects (hence, of numerical approximations and/or errors) between the initial images comprising the hollow structure H and the final representation of the device D inside said hollow structure H *(i.e.,* the deployed in-use representation R2) is minimized. This ensures the accuracy and patient-specificity of the deployed in-use representation R2 thus obtained.

Preferably, the processing unit 11 is configured to implement a finite element solver to perform this generating step S20.To this end, any finite element solver may be implemented to generate the deployed in-use representation R2. For instance, a finite element solver designed for numerically solving partial differential equations describing the interaction between wire-like structures (beam elements) against rigid surfaces may be used for generating a deployed in-use representation R2 of a vascular braided stent.

The finite element solver may be configured to provide the deployed in-use representation R2 by numerically solving frictional contact dynamics equations. Frictional contact dynamics is particularly adapted for simulating the deployment of a device D which enters in contact with tissues, *e.g.,* the inner walls of an organ or vessel.

Preferably, said same finite element solver is the same which performs the generating step S18 previously described.

In this case, each iteration of the generating step S18 allows to obtain intermediate nodal positions to which the contact forces computed by the processing unit 11 during a corresponding iteration of the generating step S20 are applied.

### Computation S201 of contact forces

More precisely, during the computation phase S201 of the generating step S20, the processing unit 11 is configured to compute contact forces between the device D and the at least one portion of the hollow structure H based on the SDF.

In other words, during this computation phase S201, the processing unit 11 (e.g., the finite element solver) may be configured to iteratively determine the presence of a contact between the device D and the hollow structure H by using a geometrical representation R1 of said device D and a hollow structure H which is implicitly described as the SDF. If the presence of a contact is confirmed, a corresponding contact force is computed S201.

The processing unit 11 may be configured to compute said contact forces by applying frictional contact dynamics equations based on the normal gap vector previously described.

The geometrical representation R1 of the device D may be a mesh comprising nodes and geometrical elements connecting said nodes. The mesh may be generated based on information of the device D such as length L1, L2 or diameter d1, d2.

For example, the geometrical representation R1 of the device D may comprise beam elements. This geometrical representation R1 is particularly adapted for a device D comprising a wire mesh. The beam elements of the geometrical representation R1 may be defined based on information on the wire mesh, such as the radius of the wires.

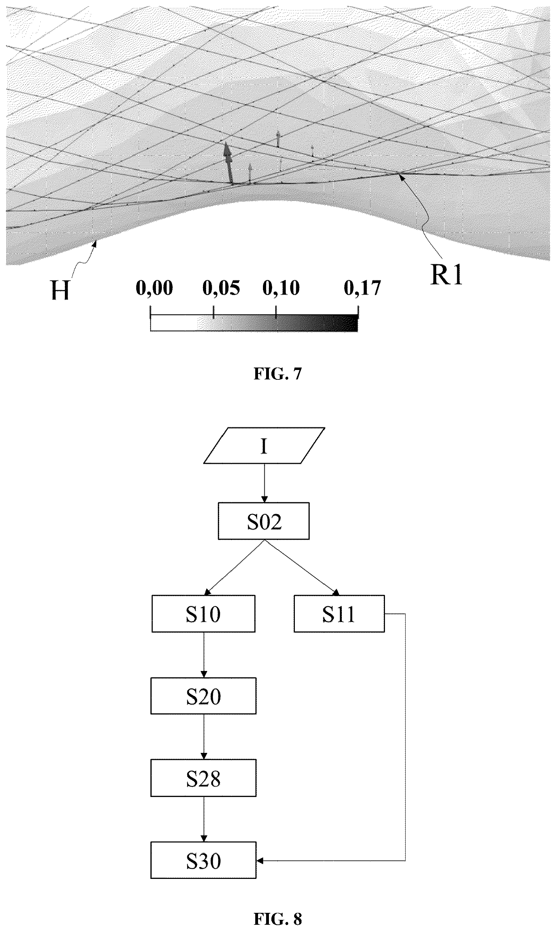

**Figure 7** illustrates an exemplary geometrical representation R1 of the device D. In this example, the geometrical representation R1 of the device D is a mesh comprising beam elements. The mesh is inside the hollow structure H (represented in grey) and the arrows represent contact forces applied to the nodes of the mesh during the contact forces application phase S202 (as will be described later). The color bar illustrates the magnitudes of said contact forces (units: N).

For example, the computation phase S201 may comprise: interpolating the SFD in a discrete number of points, and calculating the normal gap vector in these points.

The result of this interpolation of the SDF is representative of the distance between the geometrical representation R1 of the device D and the hollow structure H. In this case, the processing unit 11 is configured to compute the magnitude of the contact forces based on said distance.

By *"normal gap vector"* it is meant the vector calculated as the gradient of the SDF at a specific interpolated point. The normal gap vector has a direction normal to the surface implicitly described by the SDF at the interpolated point and a magnitude equal to the aforementioned distance. In this case, the processing unit 11 is configured to compute the direction of the contact forces based on the direction of the normal gap vector.

A contact threshold value may further be defined. For instance, the processing unit 11 may be configured to determine the presence of a contact if the aforementioned distance or the magnitude of the normal gap vector is less than said threshold value.

### Application S202 of contact forces

During the contact forces application phase S202 of the generating step S20, the processing unit 11 is configured to apply the computed contact forces to the geometrical representation R1 of the device D.

The contact forces application phase S202 may comprise using a corotational formulation for the dynamics of beam elements *(e.g.,* a corotational dynamics for frictional contact dynamics of beam elements against a surface).

During the contact forces application phase S202, the processing unit 11 may be configured to iteratively apply the calculated contact forces, for instance by calculating a kinetic energy associated with the geometrical representation R1 of the device D and applying contact forces thereto until the kinetic energy is below a predefined threshold.

**Figure 7** illustrates a portion of an exemplary geometrical representation R1 of the device D obtained at the end of an iteration of the application phase S202.

As aforementioned, the processing unit 11 may be configured to generate, during generation step S18, a deformed representation of the device D which is representative of its configuration right after implantation inside the hollow structure H.

In this case, the geometrical representation R1 of the device D to which the contact forces are applied is said deformed representation (**figure 3B**).

### Order reduction S28

As can be seen in **figure 8**, the processing unit 11 may further be configured to reduce, during an order reduction step S28, an order of the deployed in-use representation R2 of the device D so as to obtain a reduced order representation roR2 of the same.

For example, the processing unit 11 is configured to obtain the reduced order representation roR2 by applying a proper orthogonal decomposition algorithm *(e.g.,* a principal component analysis or PCA) to the deployed in-use representation R2 (also called *"high fidelity solution"* or *"snapshot")* of the device D obtained in the generating step S20 previously described, to obtain reduced bases.

In this case, the reduced order representation roR2 provides a description of the high-fidelity solution as a linear combination of a subset of reduced bases.

Moreover, the processing unit 11 may be configured to perform the order reduction S28 previously described in two distinct stages. For instance, principal features *(e.g.,* principal components, or reduced bases) and their respective projection coefficient may be calculated in a first, offline, stage, while the generation of optimal deployed configurations is performed in a second, online, stage.

The term *"online"* generally encompasses any calculation performed by the processing unit 11 as input data of said calculation are received, *i.e.,* performed in real time based on the currently received input data.

The term *"offline"* generally encompasses any calculation performed by the processing unit 11 based on previously received or acquired input data *(e.g.,* train a machine learning classifier on a stored dataset).

Such reducing step S28 comprising two decoupled stages, as described hereinabove, advantageously allows to provide a robust reduced order representation roR2, because the online stage is not dependent on the dimension of the full order model *(i.e.,* of the high-fidelity solutions, or deployed in-use representation R2), obtained in the generating step S20.

Moreover, in this case, the online stage of the reducing step S28 may be implemented by a machine learning algorithm.

Preferably, the projection coefficients are calculated during the offline stage using one of the following: Gaussian process regression, decision trees, or neural networks.

### Training S30

The processing unit 11 is configured to train, during training step S30, a machine learning system ML with each calculated geometrical parameter P as an input variable and the corresponding deployed in-use representation R2 of the device D as an associated target output.

Preferably, the input variable of the machine learning system ML is a vector of geometrical parameters (*i.e.,* a vector of machine learning features).

As aforementioned, the processing unit 11 may be configured to generate a reduced order representation roR2 of the deployed in-use representation R2.

In this case, during the training step S30, the processing unit 11 may be configured to train the machine learning system with each geometrical parameter P as an input variable and a subset of features of the reduced order representation roR2 *(e.g.,* a subset of reduced bases) as an associated target output.

The magnitude of the order reduction (for instance, the number of selected reduced bases inputted in the machine learning system) may be selected based on an error threshold. For instance, for an orthogonal decomposition, the optimal number of reduced bases (such as singular values or principal components) may be selected based on an error of said reduced bases. Such error may be derived from the singular values decay.

### Generating an optimal deployed configuration

As aforementioned, the device 1 of the present invention allows to obtain a trained machine learning system configured to generate an optimal deployed configuration of a device D inside a hollow structure H of a subject.

**Figure 9** is a schematic representation of the device 1 of the invention, showing a machine learning system ML receiving as an input at least one geometrical parameter P calculated S10 from an image dataset I acquired by an imaging apparatus 2.

In this example, the device 1 is operatively connected to the imaging apparatus 2 *(e.g.,* an MRI or a CT scanner). Alternatively, the acquired image dataset I may be received via the previously described input 10.

In this example, the device 1 of the invention advantageously comprises a graphical user interface GUI communicating with the processing unit 11. Said graphical user interface GUI allows to display the deployed configuration of a device D in real-time to a user, *e.g.,* a surgeon.

In this case, the processing unit 11 may further be configured to receive information from the user through the GUI such as, for instance, a selection of one or more seed points for initializing the segmentation S02 of the image dataset I.

Advantageously, the trained machine learning system of the invention is configured to generate the optimal deployed configuration of the device in real-time and with high accuracy.

Indeed, as can be seen in **figure 9****,** the processing unit 11 is configured to calculate, during the second calculation step S11, at least one geometrical parameter P of the hollow structure H from the image dataset I *(e.g.,* via image segmentation based on the selected points), and the trained machine learning system ML is configured to generate an optimal deployed configuration C of the device D directly therefrom.

In other words, the generation of the optimal deployed configuration C of the device D does not require the execution of steps which are time-consuming and/or which introduce errors, such as: mesh-generating algorithms, iterative solving algorithms, execution of a finite element method, SDF computation. All these steps are either avoided (as it is the case for instance for the generation of a mesh of the hollow structure H) or performed in previous steps S10, S20, S30.

Of note, the number and the complexity of the computations performed by the trained machine learning can be further reduced by implementing an order reducing step S28 comprising an offline stage and an online stage, as previously stated.

### Operation

Operation of the device 1 according to the invention will now be described.

During step S01, the processing unit 11 receives at least one image dataset I.

Then, during step S10, the processing unit 11 calculates S10 an SDF of the at least one portion of the hollow structure H on the basis of the image dataset I.

Furthermore, during step S11, the processing unit 11 calculates at least one geometrical parameter P of the portion of the hollow structure H on the basis of the image dataset I.

Alternatively, during a step S02 prior to steps S10, S11, the processing unit 11 segments the image dataset I. In this case, the at least one geometrical parameter P and/or the signed distance field SDF is calculated, during steps S10, S11, based on the segmented image dataset Is.

Then, during step S20, the processing unit 11 generates the deployed in-use representation R2 of the device D based on the previously calculated SDF.

For instance, during a contact forces computation phase S201 of the generating step S20, the processing unit 11 computes contact forces between the device D and the at least one portion of the hollow structure H based on the SDF.

Then, during a contact forces application phase S202 of the generating step S20, the processing unit 11 applies said calculated contact forces to the geometrical representation R1 of the device D.

Preferably, the contact forces are applied in the contact forces application phase S202 to a deformed representation of the device D. In this case, such deformed representation of the device D is generated by the processing unit during an optional generation step S18 prior to step S20.

Then, during step S30, the processing unit 11 trains the machine learning system ML with each calculated geometrical parameter P as an input variable and the corresponding deployed in-use representation R2 of the device D as an associated target output.

Alternatively, during step S30, the processing unit 11 trains the machine learning system ML with a reduced order representation roR2 of the device D as target output. In this case, such reduced order representation roR2 of the device D is generated during an optional order reduction step S28 prior to the training step S30.

Advantageously, the trained machine learning system generates an optimal deployed configuration of a device D inside a hollow structure H of a subject based on the at least one geometrical parameter P calculated during step S11.

### Computer-implemented method

The present invention also relates to a computer-implemented method for generating an optimal deployed configuration of a device 1 inside an organ of a subject for surgical planification, the computer program comprising program code instructions which, when executed by a computer, cause the computer to carry out the method according to any one of the embodiments described hereabove.

### Storage medium

The present invention also relates to a non-volatile computer storage medium comprising instructions which, when executed by a computer, cause the computer to carry out the steps of the method according to the invention.

Preferably, the computer-readable storage medium is a non-transitory computer-readable storage medium.

## Claims

1. A computer-implemented method for training a machine learning system configured for generating an optimal deployed configuration of a device inside a hollow structure (H) of a subject for surgical planification, said method comprising:
- based on at least one image dataset (I) representing at least one portion of the hollow structure (H):
• calculating (S10) a signed distance field (SDF) of the at least one portion of the hollow structure (H); and
• calculating (S11) at least one geometrical parameter (P) of the at least one portion of the hollow structure (H);
- generating (S20) a deployed in-use representation (R2) of the device by:
• computing (S201) contact forces between the device (D) and the at least one portion of the hollow structure (H) based on the signed distance field (SDF); and
• applying (S202) said contact forces to a geometrical representation (R1) of the device (D);
- training (S30) the machine learning system (ML) with each calculated geometrical parameter (P) as an input variable and the corresponding deployed in-use representation (R2) of the device as an associated target output, so as to obtain a trained machine learning system configured to receive as an input at least one geometrical parameter (P) and provide as output the deployed in-use representation (R2) of the device to be used in a method for generating an optimal deployed configuration of a device (D) for surgical planification.

2. The method according to claim **1,** wherein the hollow structure (H) is a blood vessel, a fistula, or a non-vascular tract of the human body.

3. The method according to claim **1** or claim **2,** further comprising segmenting (S02) the at least one image dataset (I) to obtain a segmented image dataset (Is), and wherein the at least one geometrical parameter (P) and/or the signed distance field (SDF) are calculated (S10, S11) based on the segmented image dataset (Is).

4. The method according to claim **3,** wherein the segmenting (S02) comprises applying a level set segmentation to the image dataset (I).

5. The method according to claim **4,** further comprising extracting (S03), from the segmented image dataset (Is), a three-dimensional surface representing the hollow structure (H), preferably using a marching cube algorithm.

6. The method according to any one of claims **3** to **5,** wherein the segmenting (S02) comprises an initialization phase (S021) for obtaining a zero-level set and an evolution phase (S022), said initialization phase (S021) including:
• selecting at least two points in the at least one image dataset (I);
• performing a colliding front algorithm using the at least two points as seed points.

7. The method according to any one of claims **3** to **6,** wherein the segmented image dataset (Is) comprises a set of points or a set of curves, and the calculation (S11) of the at least one geometrical parameter (P) comprises:
• applying a point set registration to the set of points; or
• applying a curve-based parametrization to the set of curves.

8. The method according to any one of claims **1** to **7,** wherein the geometrical representation (R1) of the device (D) comprises beam elements and the applying phase (S202) comprises applying a corotational formulation to the beam elements.

9. The method according to any one of claims **1** to **8,** further comprising, prior to the training (S30), reducing (S28) an order of the deployed in-use representation (R2) of the device (D) so as to obtain a reduced order representation (roR2) of the deployed device,
and wherein during the training (S30) the machine learning system is trained with the calculated geometrical parameter (P) as an input variable and a subset of features of the reduced order representation (roR2) as an associated target output.

10. The method according to claim **9,** wherein the reducing (S28) comprises: applying a proper orthogonal decomposition algorithm to the deployed in-use representation (R2) of the device.

11. The method according to any one of claims **1** to **10,** wherein the at least one geometrical parameter (P) is a centerline, a center, or a radius of the hollow structure (H).

12. The method according to any one of claims **1** to **11,** further comprising: generating (S18) a deformed representation of the device (D), the deformed representation being representative of a configuration of the device after implantation inside the hollow structure (H) of the subject, the geometrical representation (R1) of the device (D) to which contact forces are applied being said deformed representation.

13. A computer-implemented method for generating an optimal deployed configuration of a device (D) inside an organ of a subject for surgical planification, the method comprising:
- calculating (S11) at least one geometrical parameter (P) of a hollow (H) structure of the subject based on an image dataset (I) comprising at least the hollow structure (H);
- generating an optimal deployed configuration of the device by inputting the calculated at least one geometrical parameter (P) into a machine learning system trained according to the method of any one of claims **1** to **12.**

14. A device (1) for training a machine learning system configured for generating an optimal deployed configuration of a device (D) inside a hollow structure (H) of a subject for surgical planification, the device (1) comprising a processing unit (11) configured to:
- based on at least one image dataset (I) representing at least one at least one portion of the hollow structure (H):
• calculate (S10) a signed distance field (SDF) of the at least one portion of the hollow structure (H); and
• calculate (S11) at least one geometrical parameter (P) of the at least one portion of the hollow structure (H);
- generate (S20) a deployed in-use representation (R2) of the device by:
• compute (S201) contact forces between the device and the at least one portion of the hollow structure (H) based on the signed distance field (SDF); and
• apply (S202) said contact forces to a geometrical representation (R1) of the device;
- train (S30) the machine learning system with each calculated geometrical parameter (P) as an input variable and the corresponding deployed in-use representation (R2) of the device as an associated target output, so as to obtain a trained machine learning system configured to receive as an input at least one geometrical parameter (P) and provide as output the deployed in-use representation (R2) of the device to be used in a method for generating an optimal deployed configuration of a device for surgical planification.

15. A device (1) for generating an optimal deployed configuration of a device (D) inside an organ of a subject for surgical planification, the device (1) comprising a processing unit (11) configured to:
- calculate (S11) at least one geometrical parameter (P) of a hollow structure (H) of the subject based on an image dataset (I) comprising at least the hollow structure (H);
- generate an optimal deployed configuration of the device by inputting the calculated at least one geometrical parameter (P) into a machine learning system trained according to the method of any one of claims **1** to **12.**
